# EUROPEAN PATENT SPECIFICATION

(11) **EP 3 852 822 B1**
(45) Date of publication and mention of the grant of the patent: **07.06.2023**
(21) Application number: 19786867.2
(22) Date of filing: 12.09.2019
(51) Int. Cl.: A61L 27/36

(54) **PROCESS FOR THE TREATMENT OF CONNECTIVE TISSUES OF A MAMMAL**
VERFAHREN ZUR BEHANDLUNG VON BINDEGEWEBE EINES SÄUGERTIERS
PROCÉDÉ DE TRAITEMENT DE TISSUS CONJONCTIFS D'UN MAMMIFÈRE

(30) Priority: 17.09.2018 IT 201800008649
(43) Date of publication of application: 28.07.2021
(73) Proprietor: Tiss'you Srl, 47895 Domagnano (SM)
(72) Inventor: FATTORI, Paolo, 10040 Rivalta Di Torino (IT)
(74) Representative: Roncuzzi, Davide
(86) International application number: PCT/IB2019/057709
(87) International publication number: WO 2020/058813

(56) References cited:
- US-A1- 2012 009 679

## Description

The subject of the present invention is a process for the treatment of connective tissues of a mammal in the presence of:
a) a primary R-OH alcohol, wherein R is a C1-C4 linear alkyl group,
b) a hydroxide of an alkaline or alkaline earth-metal; and
c) a water scavenger agent.

The treatment of connective tissues of a mammal (tissue processing), subject of the present invention, is intended to decellularize the tissues, rupturing and removing the cells (decellularization) and the residues of the cell components from the extracellular matrix (ECM), preserving the three-dimensional structure and the biochemical/biomechanical properties of the latter, in order to produce biological scaffolds for use in regenerative medicine.

When human connective tissues are damaged, in some cases it is possible to remedy said damage via (invasive or mini-invasive) surgery, by inserting in the defect a scaffold able to cause adhesion of the host cells and allow proliferation and differentiation thereof, leading to complete recovery of the defect.

Various medical devices exist able to integrate with the human organism, and many can also be reabsorbed, leaving a completely recovered defect at the end of their function.

They can be (natural or synthetic) polymers, (reabsorbable and non-reabsorbable) metals, (natural or synthetic) inorganics or organic tissues.

Obviously the reabsorption and kinetics of these devices are strictly dependent on the type of material used. Among the tissue grafts used, autologous tissue (tissue surgically taken from the same patient) is considered the "gold standard", as it does not present any risks of rejection and it contains the patient's cells.

Obviously retrieval of the autologous tissue involves a risk at the explant stage, as the patients undergo a second surgical operation. When large defects have to be repaired, other possibilities exist in terms of biological devices: homologous tissue (tissue from a donor corpse) or heterologous tissue deriving from other animal species, in particular mammals.

To make an animal tissue compatible with the human immune system, to avoid rejections, the tissue has to be treated to eliminate the heterologous cells and their components (decellularization) or hide the antigenic epitopes (by means of cross-linking) such as, for example, the N-Terminal and C-Terminal collagen sequences (telopeptides).

Today there are many heterologous medical devices on the market, mainly of bovine and porcine origin. The producers have adopted different treatment (processing) solutions according to the connective tissue (e.g. calcination of the bone tissue or delipidization of the soft tissues with organic solvent).

Today these processes are based mainly on two principles:
1) complete elimination of the organic component of a tissue (only possible on bone tissue);
2) elimination of the cell fraction only (antigenic fraction).

Nowadays three methods are used to eliminate the cell fraction:
1) physical rupture of the cell membrane (osmotic or thermal shock);
2) solubilization of the cell membrane;
3) enzymatic hydrolysis of the cell membrane components. The clinical performances of a heterologous device depend strictly on the quality of the extracellular matrix (ECM) which the process manages to preserve. The ECM is a complex of structural proteins (e.g. collagen) and polysaccharides (e.g. glucosaminoglycans) which has well-conserved molecular sequences, also inter-species. For this reason, if a heterologous tissue is correctly processed, it represents an ideal environment for the host cells.

The methods cited above have good effectiveness in rupturing the cells, but are too aggressive vis-à-vis the extracellular matrix (e.g. thermal shock) or have action times that are penalizing for competitive production in terms of costs and productivity (enzymatic hydrolysis). Decellularization is a recognized technique that allows the use of engineered tissues deriving from ECM such as medical devices *(ECM-derived Tissue Engineered Medical Products)* in medical treatments, with reduced risk of adverse immune reaction or rejection (Crapo et al, An overview of tissue and whole organ decellularization processes, Biomaterials, 2011, 32(12), 3233).

Unlike the ECM cross-linking procedures which make the cells incorporated and the cell debris unrecognizable to the immune system, the aim of the decellularization processes is to rupture and remove the cells and/or the cell contents, preserving the structure and functionality of the ECM. More generally, decellularization is associated with a reduction in the host immune response (Keane et al, Consequences of ineffective decellularization of biologic scaffolds on the host response, Biomaterials, 2012, 33(6), 1771). The same authors have demonstrated that the effectiveness of the decellularizing agents depends on various factors, which include the cellularity of the tissue (e.g. liver vs. tendon), density (e.g. derma vs. adipose tissue), lipid content (e.g. brain vs. bladder) and thickness (e.g. derma vs. pericardium). It must be understood that every cell removal agent and method will alter the composition of the ECM, causing some degree of destruction of the ultrastructure.

The decellularization processes vary considerably in practice, using different reagents, temperatures, pressures and/or mechanical forces in parallel and/or in sequence.

The minimization of these undesired effects or the complete elimination thereof is the objective of a decellularization process.

The main agents used in decellularization processes can be: chemical (acids and bases, hypo/hypertonic solutions, detergents or organic solvents), biological (enzymes) and physical agents (temperature, pressure and electroporation).

Among the undesired effects of the agents used in the decellularization processes, the following are highlighted:
- acids and bases can damage the collagen, the glucosaminoglycans and the growth factors;
- the hypo/hypertonic solutions, the detergents and the organic solvents, used individually, have a good cell lysis capacity, but are not efficient in removing the cell components from the tissue;

- the enzymes, if exposed to the tissue at length, can degrade the ultrastructure of the extracellular matrix and remove its main constituents such as collagen, laminin, fibronectin, elastin and glucosaminoglycans;
- the physical and thermal methods can damage the structure of the extracellular matrix.

Thermal shocks, e.g. freezing by liquid nitrogen, can lead to the formation of ice crystals, mechanically rupturing the matrix. High temperatures inevitably lead to denaturation of the extracellular matrix proteins.

A chemical process has surprisingly been found able to transform, rapidly and at low production costs, the lipids present in the tissues (free lipids, phospholipids of cell membranes or lipoproteins in cells) into appropriate ester derivatives of fatty acids which compose the lipids. Said derivatives can be easily eliminated by means of washing or evaporation.

US2012/009679 A discloses a method for preparing a tissue for implantation, comprising the steps of treating a tissue material in a nuclease containing solution and with an alkaline alcohol solution. For example, the tissue may be treated with a solution comprising ethanol and sodium hydroxide.

The subject of the present invention is therefore a process for the treatment of connective tissues of a mammal in the presence of:
a) a primary R-OH alcohol, wherein R is a C1-C4 linear alkyl group,
b) a hydroxide of an alkaline or alkaline earth-metal;
c) a water scavenger agent.

Without being bound to a particular theory, it can be hypothesized that in these reaction conditions, transesterification and/or esterification of the fats and fatty acids present in the tissues takes place.

In the state of the art, the document US 2012/009679 A describes the use of an alkaline alcohol solution as follows:
*"The alcaline alcohol solution may be effective for reducing the nuclease content of the tissue, reducing the viral load of the tissue and*/*or altering the textural quality of the tissue material."*

With the above-mentioned process, the inventor targets two precise protein forms:
- Nuclease (hydrolytic enzymes that hydrolyze the phosphodiester bonds of the DNA nucleotide components);
- Prions (proteinaceous infectious particles).

The absence of a water scavenger does not allow activation of the trans-esterification reaction, which is catalysed by the alkaline component, but cannot proceed in the presence of water, since it would lead to the formation of soaps and not alkyl-esters (methanol -> methyl-esters, ethanol -> ethyl-esters, etc).

The alcohol is a primary alcohol chosen from: methanol, ethanol, propanol and butanol. The alcohol is added in a ratio of at least 3 to 1 with respect to the quantity of lipids present (see also: Helwani et al., Solid heterogeneous catalysts for transesterification of triglycerides with methanol, Applied Catalysis, A: General 363 (2009) 1-10).

The hydroxide of the alkaline or alkaline earth-metal is expediently added in a concentration ranging from 0.6 to 6% weight/weight, with respect to the total weight. Potassium hydroxide is preferred.

The water scavenger agent can be defined as a product able to remove the water and is particularly useful in synthesis reactions in which water forms as a secondary product. Removal of the water entails an increase in the production yield of the desired product, shifting the reaction equilibrium towards formation of the products. The esterification reaction is one of these reactions. The water scavenger agent can be expediently chosen from the group consisting of the drying agents such as anhydrous sodium sulphate, potassium carbonate, magnesium sulphate or silica gel etc. These solid agents are added at a concentration ranging from 1 to 10% by weight.

Or the water scavenger agent can be chosen from the group consisting of acetals having general formula (I) 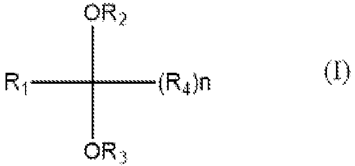 wherein
R₁ is hydrogen or a C1-C4 alkyl group;
R₂ is a C1-C4 alkyl group;
R₃ is a C1-C4 alkyl group;
R₄ is H, or a C1-C4 alkyl group or is an OR₅ alkoxy group;
R₅ is a C1-C4 alkyl group;
n varies between 1 and 2; with the condition that R₁ and R₄ are not simultaneously hydrogen.

Among the possible agents, 2,2 dimethoxypropane and trimethyl orthoformate are preferred. Said agent is added in a concentration ranging from 1 to 50 mM.

After having added the various components, the mixture is added to the tissue.

The reaction can take place at a temperature ranging from 40° to 60°C, at a pH between 8.0 and 10.0 and at a pressure ranging from 50 to 150 mbar (5kPa to 15 kPa). The reaction is carried out through to completion which is determined when the concentration of total lipids is below 0.05% out of the total weight of the (dry) tissue. The measurement is performed by analysing the residues on the tissue. The quantity of the total lipids is measured by extracting with solvent (chloroform/methanol 2:1 Folch method (1957)), evaporating the solvent, transesterifying and analysing in GC-FID the FAME according to ISO 12966.

The tissues are then rinsed in osmotic water at a temperature ranging from 40° to 60°C at atmospheric pressure, until complete removal of the reaction products and the reagents.

To eliminate the water from the product, the tissue is freeze-dried.

At the end of the process, the tissues are sterilized by means of irradiation, preferably using gamma rays.

The process, subject of the present invention, therefore comprises the following steps:
i) Delipidization and Decellularization;
ii) Washing (elimination of reaction products and reagents);
iii) Freeze-drying;
iv) Sterilization.

By means of the process of the present invention, it is possible to eliminate the lipidic components (free lipids, phospholipids of the cell membranes and lipoproteins) bringing them to a solution or gaseous phase, given their volatility, leaving the proteins in their native structure (quaternary structure) and the polysaccharides intact, since they are without free carboxylic groups that are able to react.

For this reason, the process of the present invention can be effectively applied to all connective tissues of mammals, in particular humans.

The differences of this process with respect to those currently in use are the following:
- it exploits a reaction able to transform some tissue components into reaction products that can be easily eliminated, without hydrolysing (and therefore damaging) the proteins and the polysaccharides of the ECM;
- it can be applied on all connective tissues (including musculoskeletal tissues, derma and peritoneum);
- it is the fastest method compared to the technologies currently in use (enzymatic processing = 3 weeks, whereas with the present process = 2 days);
- it is an economically advantageous method compared to the processes commonly used today.

The biological scaffolds composed of extracellular matrix (ECM) of mammal connective tissues are commonly used for the reconstruction and repair of damaged tissues.

They are produced starting from a great variety of tissues, including derma, pericardium, musculoskeletal tissue or bladder, deriving from different species of mammal (e.g. pig, cow, horse, human).

The process, subject of the present invention, is able to remedy the undesired effects of the decellularizing agents on the extracellular matrix and render uniform the treatment methods for the various connective tissues, today too sensitive to the degree of cellularization, density, lipid content and thickness of the tissue, to produce biocompatible scaffolds for the repair and reconstruction of damaged tissues.

In particular, the present invention concerns a process of trans-esterification/esterification of the free lipidic components of the tissues, the lipidic component of the lipoproteins and the phospholipidic component of the cell membranes to remove the lipidic fraction of the tissues, rupture the cells and eliminate the cell residues from the ECM.

The remaining tissue is effectively decellularized, with a residual quantity of DNA below 50 ng/mg of tissue.

The extracellular matrix maintains its macro and microstructure unchanged: collagens in their quaternary structure and 90% residual glucosaminoglycans (GAGs). Further characteristics and advantages of the invention will be more evident in the light of the detailed description of three products (biological scaffolds), as illustrated below by way of non-limiting example.

### EXPERIMENTAL PART

### Example 1

### Bone tissue

### i) Delipidization and Decellularization

A 2 cm x 2cm x 1 cm section of equine femoral epiphysis is immersed for 15 hours in a solution of methanol, KOH 0.6%, 2,2-dimethoxypropane 2 mM and stirred (100 rpm) at 60°C at a pressure of 100 mbar (10 kPa).

### ii) Washing

At the end of the reaction, the tissue is washed with osmotic water (electric conductivity < 12 µS/cm) at 60°C until complete removal of the reaction products and reagents.

### iii) Freeze-drying

The tissue is frozen at -40°C and freeze-dried at a pressure of 0.325 mbar increasing the temperature by 10°C/h up to a final temperature of 20°C.

### iv) Sterilization

The packaged product is sterilized by means of gamma irradiation, at 25 kGy.

The end product of the above-mentioned processing, analysed at a specialist centre, has a mineral composition which is unchanged compared to the non-treated product, both in its chemical formula and in its crystallographic structure.

The protein composition shows complete elimination of the cell proteins and no change in those belonging to the ECM.

The result of the analysis performed is the following: "The crystallographic analysis shows maintenance of the crystallographic structure of the calcium hydroxyapatite. The identification analysis of the proteins present in the tissues treated shows complete absence of cell proteins and preservation of the extracellular matrix proteins."

### Example 2

### Derma

### i) Delipidization and Decellularization

A 20 cm x 30 cm section of porcine derma is immersed for 6 hours in a solution of methanol, NaOH 4%, 5% anhydrous sodium sulphate and stirred (100 rpm) at 40°C at a pressure of 50 mbar.

### ii) Washing

At the end of the reaction, the tissue is washed with osmotic water (electric conductivity < 12 µS/cm) at 40°C until complete removal of the reaction products and reagents.

### iii) Freeze-drying

The tissue is frozen at -40°C and freeze-dried at a pressure of 0.325 mbar, increasing the temperature by 10°C/h up to a final temperature of 20°C.

### iv) Sterilization

The packaged product is sterilized by means of gamma irradiation, at 25 kGy.

The end product of the processing, analysed at a specialist centre, is decellularized with a residual DNA concentration of 20 ng/mg. The protein composition shows complete elimination of the cell proteins and no change in those belonging to the ECM. The concentration of GAGs is 90% of the initial concentration.

The result of the analysis performed is the following: "The biochemical analysis of the heterologous derma shows decellularization of the tissue, measured with the residual DNA method. Maintenance of the extracellular matrix was verified via the residual concentration of glucosaminoglycans. The tissue is decellularized and its extracellular matrix is preserved".

### Example 3

### Pericardium

### i) Delipidization and Decellularization

A circular section of 18 cm in diameter of bovine pericardium is immersed for 6 hours in a solution of ethanol, KOH 6%, 2,2-dimethoxypropane 50 mM and stirred (100 rpm) at 60°C at a pressure of 50 mbar.

### ii) Washing

At the end of the reaction, the tissue is washed with osmotic water (electric conductivity < 12 µS/cm) at 40°C until complete removal of the reaction products and reagents.

### iii) Freeze-drying

The tissue is frozen at -40°C and freeze-dried at a pressure of 0.325 mbar, increasing the temperature by 10°C/h up to a final temperature of 20°C.

### iv) Sterilization

The packaged product is sterilized by means of gamma irradiation, at 25 kGy.

The end product of the processing, analysed at a specialist centre, is decellularized with a residual DNA concentration of 40 ng/mg. The protein composition shows complete elimination of the cell proteins and no change in those belonging to the ECM. The concentration of GAGs is 95% of the initial concentration.

The result of the analysis performed is the following: "The biochemical analysis of the heterologous pericardium shows decellularization of the tissue, measured with the residual DNA method. Maintenance of the extracellular matrix was verified via the residual concentration of glucosaminoglycans. The tissue is decellularized and its extracellular matrix is preserved".

## Claims

1. A process for the ex vivo treatment of connective tissues of a mammal in the presence of:
a) a primary R-OH alcohol, wherein R is a C1-C4 linear alkyl group;
b) a hydroxide of an alkaline or alkaline-earth metal; and
c) a water scavenger agent.

2. The process according to claim 1 in which the mammal is a human.

3. The process according to claim 1 and 2, wherein the alcohol is a primary alcohol selected from: methanol, ethanol, propanol and butanol added in a 3: 1 ratio with respect to the tissue.

4. The process according to any of claims 1 to 3, in which the alkaline or alkaline-earth-metal hydroxide is added in a concentration between 0.6 and 6% wt/wt, with respect to the total weight.

5. The process according to any of the claims 1-4, wherein the water scavenger agent is selected from the group consisting of the drying agents; in a concentration ranging from 1 to 10% by weight; or the water scavenger agent can be selected from the group consisting of acetals having general formula (I) 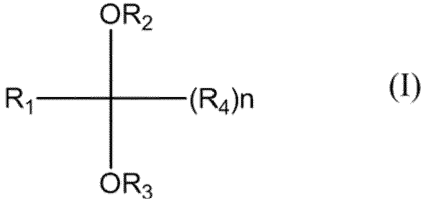 wherein
R₁ is hydrogen or a C1-C4 alkyl group;
R₂ is a C1-C4 alkyl group;
R₃ is a C1-C4 alkyl group;
R₄ is H, or a C1-C4 alkyl group or is an alkoxy group OR₅;
R₅ is a C1-C4 alkyl group;
n ranges between 1 and 2;
with the condition that R₁ and R₄ are not hydrogen at the same time; and are in a concentration between 1 and 50 mM.

6. The process according to any of claims 1-5, wherein the alcohol is methanol or ethanol; the alkaline or alkaline-earth-metal hydroxide is potassium hydroxide; the water scavenger agent is sodium sulphate or 2,2 - dimethoxypropane.

7. The process according to each of the claims 1-6, carried out at a temperature ranging from 40°C to 60°C, at a pH between 8.0 and 10.0 and at a pressure of between 50 and 150 mbar (5kPa to 15 kPa).

## Patentansprüche

1. Ein Verfahren für die Ex-vivo-Behandlung von Bindegeweben eines Säugetiers in Gegenwart von Folgendem:
a) einem primären R-OH-Alkohol, wobei R eine lineare C1-C4-Alkylgruppe ist;
b) einem Hydroxid eines Alkali- oder Erdalkalimetalls; und
c) einem Wasser-Scavengermittel.

2. Verfahren gemäß Anspruch 1, in dem das Säugetier ein Mensch ist.

3. Verfahren gemäß den Ansprüchen 1 und 2, wobei der Alkohol ein primärer Alkohol ist, der aus Folgendem ausgewählt ist: Methanol, Ethanol, Propanol und Butanol, hinzugefügt in einem 3:1-Verhältnis in Bezug auf das Gewebe.

4. Verfahren gemäß einem der Ansprüche 1 bis 3, in dem das Alkali- oder Erdalkalimetall-Hydroxid in einer Konzentration zwischen 0,6 und 6 % Gew./Gew., in Bezug auf das Gesamtgewicht, hinzugefügt wird.

5. Verfahren gemäß einem der Ansprüche 1-4, wobei das Wasser-Scavengermittel aus der Gruppe ausgewählt ist, die aus den Trocknungsmitteln besteht; die in einer Konzentration im Bereich von 1 bis 10 Gewichts-% vorliegen; oder das Wasser-Scavengermittel aus der Gruppe ausgewählt werden kann, die aus Acetalen besteht, die die folgende allgemeine Formel (I) aufweisen: 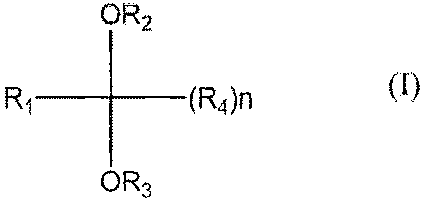 wobei
R₁ Wasserstoff oder eine C1-C4-Alkylgruppe ist;
R₂ eine C1-C4-Alkylgruppe ist;
R₃ eine C1-C4-Alkylgruppe ist;
R₄ H ist oder eine C1-C4-Alkylgruppe ist oder eine Alkoxygruppe OR₅ ist;
R₅ eine C1-C4-Alkylgruppe ist;
n im Bereich zwischen 1 und 2 liegt;
mit der Bedingung, dass R₁ und R₄ nicht gleichzeitig Wasserstoff sind; und die in einer Konzentration zwischen 1 und 50 mM vorliegen.

6. Verfahren gemäß einem der Ansprüche 1-5, wobei der Alkohol Methanol oder Ethanol ist; das Alkali- oder Erdalkalimetall-Hydroxid Kaliumhydroxid ist; das Wasser-Scavengermittel Natriumsulfat oder 2,2-Dimethoxypropan ist.

7. Verfahren gemäß jedem der Ansprüche 1-6, das bei einer Temperatur im Bereich von 40 °C bis 60 °C, bei einem pH-Wert zwischen 8,0 und 10,0 und bei einem Druck von zwischen 50 und 150 mbar (5 kPa bis 15 kPa) ausgeführt wird.

## Revendications

1. Un procédé pour le traitement ex vivo de tissus conjonctifs d'un mammifère en présence de :
a) un alcool R-OH primaire, où R est un groupe alkyle en C1-C4 linéaire ;
b) un hydroxyde d'un métal alcalin ou alcalino-terreux ; et
c) un agent capteur d'eau.

2. Le procédé selon la revendication 1 dans lequel le mammifère est un être humain.

3. Le procédé selon la revendication 1 et la revendication 2, où l'alcool est un alcool primaire sélectionné parmi : le méthanol, l'éthanol, le propanol et le butanol ajouté à raison d'un rapport de 3/1 relativement au tissu.

4. Le procédé selon n'importe lesquelles des revendications 1 à 3, dans lequel l'hydroxyde de métal alcalin ou alcalino-terreux est ajouté à une concentration comprise entre 0,6 et 6 % p/p, relativement au poids total.

5. Le procédé selon n'importe lesquelles des revendications 1 à 4, où l'agent capteur d'eau est sélectionné dans le groupe constitué des agents desséchants ; dans une concentration allant de 1 à 10 % en poids ; ou l'agent capteur d'eau peut être sélectionné dans le groupe constitué d'acétals ayant la formule générale (I) 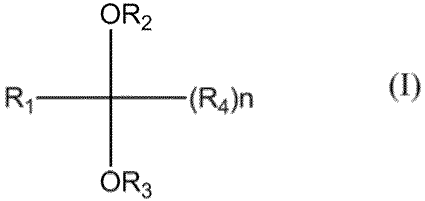 où
R₁ est l'hydrogène ou un groupe alkyle en C1-C4 ;
R₂ est un groupe alkyle en C1-C4 ;
R₃ est un groupe alkyle en C1-C4 ;
R₄ est H, ou un groupe alkyle en C1-C4 ou est un groupe OR₅ alcoxy ;
R₅ est un groupe alkyle en C1-C4 ;
n va de 1 à 2 ;
à la condition que R₁ et R₄ ne soient pas l'hydrogène en même temps ; et soient dans une concentration comprise entre 1 et 50 mM.

6. Le procédé selon n'importe lesquelles des revendications 1 à 5, où l'alcool est le méthanol ou l'éthanol ; l'hydroxyde de métal alcalin ou alcalino-terreux est l'hydroxyde de potassium ; l'agent capteur d'eau est le sulfate de sodium ou le 2,2-diméthoxypropane.

7. Le procédé selon chacune des revendications 1 à 6, mené à une température allant de 40 °C à 60 °C, à un pH compris entre 8,0 et 10,0 et à une pression comprise entre 50 et 150 mbar (5 kPa à 15 kPa).
